# EUROPEAN PATENT SPECIFICATION

(11) **EP 1 842 914 B1**
(45) Date of publication and mention of the grant of the patent: **06.10.2010**
(21) Application number: 06124822.5
(22) Date of filing: 27.11.2006
(51) Int. Cl.: C12N 15/10, C12Q 1/68

(54) **Method and apparatus for disrupting cells and purifying nucleic acid using a single chamber**
Verfahren und Vorrichtung zum Aufbrechen von Zellen und zur Aufreinigung von Nukleinsäure in einer einzelnen Kammer
Procédé et dispositif de disruption de cellules et de purification d'acide nucléique dans une chambre unique

(30) Priority: 05.04.2006 KR 20060030990
(43) Date of publication of application: 10.10.2007
(73) Proprietor: Samsung Electronics Co., Ltd., Suwon-si, Gyeonggi-Do (KR)
(72) Inventor: Lee, In-ho c/o Samsung Advanced Institute of Technology, Gyeonggi-do (KR); Han, Ki-woong c/o Samsung Advanced Institute of Technology, Gyeonggi-do (KR); Lee, Jeong-gun c/o Samsung Advanced Institute of Technology, Gyeonggi-do (KR); Yoo, Chang-eun c/o Samsung Advanced Institute of Technology, Gyeonggi-do (KR); You, Jae-ho c/o Samsung Advanced Institute of Technology, Gyeonggi-do (KR); Kim, Young-rok c/o Samsung Advanced Institute of Technology, Gyeonggi-do (KR); Min, Jun-hong c/o Samsung Advanced Institute of Technology, Gyeonggi-do (KR)
(74) Representative: Grünecker, Kinkeldey, Stockmair & Schwanhäusser Anwaltssozietät

(56) References cited:
- EP-A2- 1 650 297
- WO-A-01/46404
- US-A1- 2003 096 429
- HOFMANN OLIVER ET AL: "Laser induced disruption of bacterial spores on a microchip" LAB ON A CHIP, vol. 5, no. 4, 23 February 2005 (2005-02-23), pages 374-377, XP002417817 ISSN: 1473-0189
- LEE JEONG-GUN ET AL: "Microchip-based one step DNA extraction and real-time PCR in one chamber for rapid pathogen identification" LAB ON A CHIP, vol. 6, no. 7, 2006, pages 886-895, XP002417818 ISSN: 1473-0197(print) 1473-0189(ele
- ROBIN HUI LIU ET AL: "SELF-CONTAINED, FULLY INTEGRATED BIOCHIP FOR SAMPLE PREPARATION, POLYMERASE CHAIN REACTION AMPLIFICATION, AND DNA MICROARRAY DETECTION" ANALYTICAL CHEMISTRY, AMERICAN CHEMICAL SOCIETY. COLUMBUS, US, vol. 76, no. 7, 1 April 2004 (2004-04-01), pages 1824-1831, XP001196720 ISSN: 0003-2700

## Description

### BACKGROUND OF THE INVENTION

### 1. Field of the Invention

The present invention relates to a chip for isolating nucleic acids from cells or viruses and a method and an apparatus for disrupting cells and purifying nucleic acid using a single chamber.

### 2. Description of the Related Art

Methods of isolating DNA from cells are performed using materials that have a proclivity of binding to DNA. Examples of materials used for DNA isolation include silica, glass fiber, anion exchange resin and magnetic beads (Rudi, K. et al., Biotechniqures 22, 506-511 (1997); and Deggerdal, A. et al., Biotechniqures 22, 554-557 (1997)). To avoid manual operation and remove operator error, several automatic machines have been developed for high-throughput DNA extraction.

The production of high purity double-stranded plasmid DNAs, single-stranded phage DNAs, chromosomal DNAs, and agarose gel-purified DNA fragments is very important in molecular biology. Ideal methods of purifying DNAs should be simple and quick, and include little additional manipulation of samples. The DNAs obtained using such methods are ready for direct transformation, restriction enzyme analysis, ligation or sequencing. Such methods are very attractive in the automated production of DNA samples, which is favored in research and diagnosis labs.

Conventionally, a method of purifying nucleic acids using a solid phase is known. For example, U.S. Patent No. 5,234,809 discloses a method of purifying nucleic acids using a solid phase to which nucleic acids are bound, the method including: mixing a starting material, a chaotropic material, and a nucleic acid binding solid phase; separating the solid phase with the nucleic acid bound thereto from the liquid, and washing the solid phase nucleic acid complexes. However, this method is time consuming and complicated, and thus is not suitable for a Lab-On-a-Chip (LOC). Also, the method has a problem in that a chaotropic material should be used.

U.S. Patent No. 6,291,166 discloses a method of archiving nucleic acids using a solid phase matrix. This method is advantageous in that a delayed analysis or repeated analysis for the nucleic acid solid phase matrix complexes after storage is possible since nucleic acids are irreversibly bound to the solid phase matrix. However, according to this method, alumina (Al₂O₃) which has a positively-charged surface needs to be rendered hydrophilic by basic materials, such as NaOH, and nucleic acids are irreversibly bound to the alumina rendered hydrophilic, and thus cannot be separated from the alumina.

US Patent No. 6,936,414 discloses a method of separating nucleic acid from a test sample, the method comprising: contacting a test sample with a metal oxide support material and a binding buffer to form nucleic acid/metal oxide support material complexes; separating the complexes from the test sample; and eluting the nucleic acid from the metal oxide support material. However, although the invention disclosed in US Patent No. 6,936,414 is similar to the present invention in that binding of metal oxide and nucleic acid is used, the invention disclosed in US Patent No. 6,936,414 is different from the present invention in that the invention disclosed in US Patent No. 6,936,414 uses a chaotropic salt and a detergent as binding materials when nucleic acids are bound to the metal oxide support, but the present invention does not.

EP-A2-1650297 relates to a method and an apparatus for rapid disruption of cells or viruses using micro magnetic beads added to a solution containing cells or viruses and irradiating laser light onto the magnetic beads to disrupt the cells or viruses.

US 2003/096429 A1 refers to a laser induced cell lysis system and a method of determining laser parameters for cell lysis.

Hofmann, Oliver et al. "Laser induced disruption of bacterial spores on a microchip", Vol. 5, No. 4, February 23, 2005, pages 374-377 relates to a laser based disruption of Bacillus spores on a microchip. The spores are mixed with a laser light-absorbing matrix and the mixture is then co-crystallized in nanovials. After attaching a flow cell with a micro-structured channel to the target plate, the sample zone of co-crystallized spores and matrix is irradiated with pulsed UV laser light effecting a disintegration of matrix with concomitant disruption of the outer spore layer.

The inventors of the present invention studied methods of disrupting cells and purifying nucleic acid based on conventional techniques described above, and confirmed that a Lab-On-a-Chip integration could be achieved, by developing a technique of performing cell enrichment, cell lysis, and a process of purifying nucleic acids that are required in a Lab-On-a-Chip technique in a single chamber, and thus completed the present invention.

### SUMMARY OF THE INVENTION

The present invention provides a method of disrupting cells or viruses and purifying nucleic acid, the method comprising:
adding a cell-containing or virus-containing solution to a solid support onto which a lysis enhancement metal oxide capable of enhancing cell or virus lysis and a binding metal oxide capable of binding a) cells or viruses, and b) nucleic acid are sequentially deposited for binding the cells or viruses to the deposited binding metal oxide, wherein the lysis enhancement metal oxide is Fe₂O₃ and the binding metal oxide is Al₂O₃;
irradiating a laser beam onto the binding metal oxide for disrupting the cells or viruses bound to the binding metal oxide, whereupon nucleic acids released from the lysed cells or viruses are bound to the binding metal oxide; and
washing off the unbound cell or virus components using a washing buffer.

The present invention also provides a chip for disrupting cells or viruses and purifying nucleic acids comprising a solid support onto which a lysis enhancement metal oxide enhancing cell or virus lysis and a binding metal oxide binding a) cells or viruses, and b) nucleic acid have been sequentially deposited, wherein the lysis enhancement metal oxide is Fe₂O₃ and the binding metal oxide is Al₂O₃.

The present invention also provides an apparatus for disrupting cells and purifying nucleic acids, the apparatus comprising:
a chip according to the preceding paragraph; and
a laser generating means.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates a method of concentrating cells and purifying nucleic acids in a single chamber according to an embodiment of the present invention;
FIG. 2 is a view illustrating a chip comprising a solid support having a pillar structure on which metal oxide is deposited according to an embodiment of the present invention;
FIG. 3A illustrates an upper portion, a middle portion and a lower portion of a chip on which a cell lysis metal oxide enhancing layer was deposited;
FIG. 3B is a series of field emission-scanning electron microscope (FE-SEM) images of the chip of FIG. 3A taken in each position described above;
FIG. 4 is a FE-SEM image showing binding efficiency of *E. coli* cells according to different kinds of chips;
FIG. 5 is a FE-SEM image showing *E. coli* cells lysis according to different kinds of chips;
FIG.6 is a graph showing eluting efficiency of *E. coli* genome DNA (gDNA) with respect to washing buffers and nucleic acid eluting buffers;
FIG. 7 is a graph is a graph illustrating a quantification curve of eluted DNA with respect to concentration of input *E. coli* cells; and
FIG. 8 is a graph illustrating a quantification curve of a PCR product with respect to concentration of input *E. coli* cells.

### DETAILED DESCRIPTION OF THE INVENTION

The present invention will now be described more in detail with reference to the accompanying drawings, in which exemplary embodiments of the invention are shown.

The present invention provides a method of disrupting cells or viruses and purifying nucleic acids in a single chip, the method comprising: irradiating laser to a chip in which a cell lysis enhancing metal oxide, and a cell or virus and nucleic acid binding metal oxide are sequentially deposited on a solid support.

In the method according to an embodiment of the present invention, cell or virus disruption and nucleic acid purification are processed on one single chip. When a laser beam is irradiated to the chip on which a cell lysis enhancing metal oxide is deposited first, and after that a metal oxide that can bind cells or viruses and nucleic acids is deposited, cells or viruses are lysed and subsequently nucleic acids can be purified from the lysed cells or viruses on the single chip.

The present invention also provides a method of disrupting cells or viruses and purifying nucleic acids using a single chamber, the method comprising: sequentially depositing a cell lysis enhancing metal oxide, and a cell or virus and nucleic acid binding metal oxide on a solid support; adding a cell-containing or virus-containing solution to the solid support on which the cell lysis enhancing metal oxide, and the cell or virus and nucleic acid binding metal oxide are deposited to bind the cell or virus with the deposited cell or virus and nucleic acid metal oxide; disrupting the cell or virus bound to the cell or virus and nucleic acid binding metal oxide by irradiating a laser beam to bind a nucleic acid with the cell or virus and nucleic acid binding metal oxide; washing a test sample that is not bound to the cell or virus and nucleic acid binding metal oxide using a washing buffer; and eluting the nucleic acid bound to the cell or virus and nucleic acid binding metal oxide by adding a nucleic acid eluting buffer.

In an embodiment of the method according to the present invention, cell concentration, cell lysis and nucleic acid purification in a single chamber are possible. In conventional methods, many steps from cell concentration to nucleic acid purification are required, chambers are needed in each step, and many valves and pumps are required. Therefore, the known processes can not be performed in a single chamber.

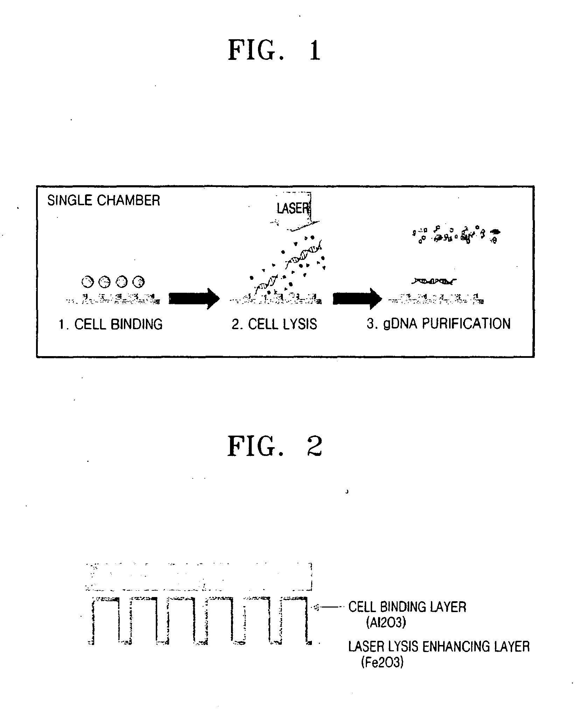

FIG. 1 illustrates a method of concentrating and disrupting cells, thereby purifying nucleic acid in a single chamber, according to an embodiment of the present invention. Referring to FIG. 1, the surface of a support having a pillar structure is treated with a metal oxide having a hydrophobic property for binding cells or viruses, and thus cells are concentrated on the support. Subsequently, by absorbing laser light having a particular wavelength, the concentrated cells are improvably lysed due to the application of a cell lysis enhancing layer onto the pillar structure. The lysed cells are then further processed for nucleic acid purification using a washing buffer and an eluting buffer. All the processes described above are performed in a single chamber.

According to an embodiment of the present invention, metal oxide is deposited on a solid support, and then a cell-containing or virus-containing solution is added thereto, and thus cells or viruses are bound to the solid support. Then, a laser beam is irradiated to the bound cells or viruses, and thus the cells or viruses bound to the solid support are disrupted and nucleic acids eluted by the disruption of the cells or viruses are bound to the solid support. Subsequently, impurities such as cell debris and protein that are not bound to the solid support are washed off using a washing buffer. Then the nucleic acid bound to the solid support is eluted by adding a nucleic acid eluting buffer. Finally, the eluted nucleic acid can be used in the subsequent process.

In the method according to the current embodiment of the present invention, metal oxide deposition on the solid support is performed by first depositing a cell lysis enhancing metal oxide, and then depositing a metal oxide to which cell binding is possible thereon. The cell lysis enhancing metal oxide is a material forming a layer that can enhance cell lysis if irradiated by a laser beam, i.e. achieving a similar function to that of a magnetic bead performing cell lysis, and is the metal oxide Fe₂O₃. Cell binding is possible with a metal oxide having the proclivity of cell binding. The cell binding metal oxide is Al₂O₃. The metal oxide for cell binding should be transparent such that a laser beam can pass through the cell binding metal oxide and reach the cell lysis enhancing metal oxide. Further, the metal oxide for cell binding can bind the nucleic acid of cells or viruses disrupted by the laser beam. FIG. 2 is a view illustrating a solid support on which a metal oxide is deposited according to an embodiment of the present invention, that is, a chip having a pillar structure. In FIG. 2, Fe₂O₃ is used as the cell lysis enhancing metal oxide, and Al₂O₃ is used as the binding metal oxide to which cell binding is possible. Fe₂O₃ absorbs laser beams having a wavelength of 808 nm, thereby enhancing cell lysis.

The cell or viruses and nucleic acid binding metal oxide is Al₂O₃ and the cell lysis enhancing metal oxide is Fe₂O₃.

In the method according to the current embodiment of the present invention, the solid support can be glass slide, a silicon wafer, a magnetic bead, polystyrene, a membrane, a metal plate, or the like, but is not limited thereto. The solid support can be any water-insoluble support material on which metal oxide can be deposited. When a solid support is soluble in water, it is difficult to separate a nucleic acid solution from the solid support after the nucleic acid is purified. In addition, use of a solid support having a large surface area is desirable because more metal oxide can be deposited thereon. Therefore, in case of a plane support material, such as glass slide or a wafer, the surface of the plane support material can be processed to have a pillar structure to increase its surface area.

In the method according to the current embodiment of the present invention, the cell-containing or virus-containing solution may have a pH of 3 -10. When the pH of the cell-containing or virus-containing solution is outside this range, the efficiency of binding the cell or the nucleic acid to the solid support onto which the cell or virus and nucleic acid binding metal oxide is deposited decreases, and DNA can be physically and chemically denatured. Therefore, the subsequent process can be negatively affected.

In the method according to the current embodiment of the present invention, the washing buffer may have a pH of 3 -10. When the pH of the washing buffer is outside this range, the efficiency of binding the nucleic acid to the solid support on which a metal oxide is deposited decreases, and DNA can be physically and chemically denatured. Therefore, the subsequent process can be hampered.

In the method according to the current embodiment of the present invention, the cell-containing or virus-containing solution, the washing buffer and the nucleic eluting buffer can be phosphate buffer, acetate buffer, citrate buffer, Tris buffer, sulfate buffer, and the like, without being limited thereto.

In the method according to the current embodiment of the present invention, the concentration of the cell-containing or virus-containing solution may be in the range of 10 -1,000 mM. When the buffer concentration of the cells or viruses in the cell-containing or virus-containing solution is less than 10 mM, the efficiency of binding the cell or virus to the solid support on which the cell or virus and nucleic acid binding metal oxide is deposited decreases. When the concentration of the cells or viruses in the cell-containing or virus-containing solution is greater than 1,000 mM, it is difficult to prepare a solution.

In the method according to the current embodiment of the present invention, the concentration of the washing buffer may be 10-1,000 mM. When the concentration of the washing buffer is less than 10 mM, the efficiency of washing off impurities such as cell debris or proteins decreases. When the concentration of the washing buffer is greater than 1,000 mM, it is difficult to prepare a solution.

In the method according to the current embodiment of the present invention, the nucleic acid eluting buffer may have a pH of 8-10. When the pH of the nucleic acid eluting buffer is less than 8, the efficiency of eluting the nucleic acid from the solid support on which the cell or virus and nucleic acid binding metal oxide is deposited decreases. When the pH of the nucleic acid eluting buffer is greater than 10, the subsequent process can be hampered.

In the method according to the current embodiment of the present invention, the concentration of the nucleic acid eluting buffer may be 10-100 mM. When the concentration of the nucleic acid eluting buffer is outside this range, the efficiency of eluting the nucleic acid bound to a solid support on which a metal oxide is deposited decreases, and also the subsequent process can be negatively affected.

In the method according to the current embodiment of the present invention, the metal oxide may be deposited by physical vapor deposition (PVD), atomic layer deposition (ALD), sol-gel method, or the like. Generally, metal oxide can be deposited on a solid support using a known technique, such as PVD, ALD, sol-gel method, or the like.

PVD is a method that is used in thin film formation, and has recently been favored as a means of surface curing, since a thin film can be relatively simply obtained by low temperature treatment that cannot be applied when using other deposition methods. Examples of PVD methods include an evaporation deposition method that does not use ions, a sputtering method that uses ions, an ion plating method, an ion implantation method, an ion beam mixing method and the like. Recently, the use of the ion plating method, the ion implantation method, the ion beam mixing method, etc. has been favored, since a proper film can be formed at low temperature efficiently using ion energy. Metal is evaporated when heated in a vacuum, and this principle is applied in the evaporation deposition method. This method is performed under high vacuum conditions of less than 10⁻⁵ Torr and uses metal or one of a variety of compounds as a coating material. The method is applied in optical elements such as lenses, mirrors or the like, all kinds of electric elements and plastic elements, but it is barely used for surface curing. Through the PVD ion plating method, a hard film that has the best adhesion and a thickness of more than microns (µm) can be obtained, so that the method can be also applied to fields such as tools or molds, etc. When a particle having high energy is collided with a target material, atoms or molecules are ejected from the target material, and this phenomenon is called sputtering. In sputtering, a target material and a substrate form an anode and a cathode, respectively, and then a high pressure of about 10⁻² Torr is applied between the anode and cathode under Ar atmosphere, and as a result, Ar gas around the anode is ionized to be Ar⁺ and then the Ar⁺ is collided with the anode. Molecules or atoms ejected by the ion bombardment are bound to the substrate, that is, the cathode, to form a thin film. Examples of sputtering include DC sputtering, RF sputtering, bias sputtering, magnetron sputtering and the like, and in particular, magnetron sputtering is a high speed sputtering method and favored in various fields.

In the ALD method, a molecule is absorbed into a wafer surface using a chemically sticking property. The absorbed molecule is subsequently substituted. Since absorption and substitution are alternatively performed, ultrafine layer-by ultrafine layer deposition is possible, and an oxide and a thin metal film can be stacked as thin as possible. Also, a suitable film can be formed at a temperature (less than 500 °C) than lower the temperature used in the chemical vapor deposition (CVD) method, so that the ALD method is suitable for System-on-Chip (SoC) manufacture.

In the sol-gel method, a metal oxide having a colloid form is prepared by means of a hydrolysis reaction of a metal halide or alkoxide. The sol-gel method is a representative method of preparing a coating solution of titanium dioxide (Ti02). Physical characteristics of the titanium dioxide prepared, such as a particle size, crystalline property and the like are affected by the kind of alkoxide used, reaction conditions (temperature, pH, a mol ratio between reactants, etc.) and the like.

In the method according to the current embodiment of the present invention, the process in which a cell or a virus is bound to the solid support and the nucleic acid eluting process can be performed under static or fluidic conditions. Contacting a cell or a virus with the solid support is possible under static conditions, and also under fluidic conditions. That is, a cell or a virus is contacted with a solid support by flowing a solution containing a cell or a virus in a fluidic control system. In the fluidic control system, the solid support can have a plane form, but may have a pillar structure in order to bind more cells or viruses by increasing the probability of contacting the solid support with the cells or viruses.

In the method according to the current embodiment of the present invention, a process in which a nucleic acid is eluted from the solid support and then the eluted nucleic acid is detected can be additionally included. The eluted nucleic acid can be detected using an electrophoretic method, a nucleotide sequencing, a restriction enzyme analysis or the like.

In the method according to the current embodiment of the present invention, a method step in which isolated nucleic acid is eluted from the solid support and then the eluted nucleic acid is amplified can be additionally be carried out. When the amount of the eluted nucleic acid is so small that its direct detection is not possible, the eluted nucleic acid can be amplified using a PCR method, so that the amplified nucleic acid can be easily detected.

In the method according to the current embodiment of the present invention, the nucleic acid amplification process can be performed without removing, i.e. in the presence of the nucleic acid eluting buffer. The nucleic acid eluting buffer has a very similar composition to that of the buffer used for the nucleic acid amplification, so that the nucleic acid eluted in the eluting buffer can be immediately amplified without the need to remove the eluting buffer.

In the method according to the current embodiment of the present invention, the laser beam can be irradiated as a laser pulse or a continuous laser wave. The laser pulse should have an output of more than 1 mJ/pulse, and the continuous laser wave should have an output of more than 10 mW. Preferably, the output of the laser pulse is more than 3 mJ/pulse, and the output of the continuous laser wave is more than 100 mW. When the output of the laser pulse is less than 1 mJ/pulse and the output of the continuous laser wave is less than 10 mW, the laser beams cannot transfer enough energy to disrupt cells, which can be a problem.

In the method according to the current embodiment of the present invention, the laser may generate a laser beam having a wavelength in the range of more than 400 nm, and preferably in the range form 750 nm-1,300 nm. When the laser beam has a wavelength range of less than 400 nm, DNA can be denatured or damaged. Further, the laser can generate a laser beam of at least one wavelength range, that is, the laser may generate radiation have one wavelength within the wavelength range described above, and may have more than two different wavelengths.

According to another embodiment of the present invention, there is provided a chip wherein cell lysis enhancing metal oxide, and cell or virus and nucleic acid binding metal oxide are sequentially deposited on a solid support.

According to the current embodiment of the present invention, the chip is designed to perform a process of cell or virus lysis and nucleic acid purification on the chip. On the chip, the cell lysis enhancing metal oxide, via which cell lysis by irradiation with a laser beam is enhanced, is first deposited, and subsequently a metal oxide that can bind cells or viruses and nucleic acids is deposited thereon. Therefore, in a single chip, cells or viruses can be lysed, and subsequently the nucleic acids can be isolated and purified from the lysed cells or viruses.

The solid support may preferably have a large surface area for more metal oxide deposition thereon. In case of plane support such as glass slide or a wafer, the surface thereof can be processed to have a pillar structure to increase the surface area. Accordingly, the solid support can have a plane or pillar structure.

In the chip according to the current embodiment of the present invention, the metal oxide that can bind cells or viruses and nucleic acids is Al₂O₃ and the cell lysis enhancing metal oxide is Fe₂O₃.

According to another embodiment of the present invention, an apparatus for disrupting cells and purifying nucleic acids includes a single chamber comprises a chip, wherein a cell lysis enhancing metal oxide and a cell or virus and nucleic acid binding metal oxide are sequentially deposited on a solid support, and a laser generating means.

The apparatus for disrupting cells and purifying nucleic acids according to the current embodiment of the present invention comprises a chip that reversibly binds cells or viruses and nucleic acids so that the nucleic acids can be eluted from the chip. The apparatus further comprises a laser generating means that provides a laser beam to disrupt the cells or viruses bound to the chip. In the apparatus, disrupting cells or viruses and purifying nucleic acids is performed in a single chamber.

In the apparatus according to the current embodiment of the present invention, the solid support can be glass slide, a silicon wafer, a magnetic bead, polystyrene, a membrane, a metal plate or the like, without being limited thereto. The solid support can be any material on which metal oxide can be deposited, but should be insoluble in water. When the solid support is soluble in water, it is difficult to separate the solid support from a nucleic acid solution after nucleic acid purification. Also, the solid support may have a large surface area so that a plurality of metal oxides can be deposited thereon. In case of a plane support material such as glass slide or a wafer, its surface can be processed to have a pillar structure in order to increase the surface area. Accordingly, the solid support can have a plane or pillar structure.

In the apparatus according to the current embodiment of the present invention, the metal oxide that can bind cells or viruses and nucleic acids is Al₂O₃, and the cell lysis enhancing metal oxide is Fe₂O₃.

In the apparatus according to the current embodiment of the present invention, the laser can be a pulse laser or a continuous wave laser. The pulse laser should have an output of more than 1 mJ/pulse, and the continuous wave laser should have an output of more than 10 mW. Preferably, the output of the pulse laser is more than 3 mJ/pulse, and the output of the continuous wave laser is more than 100 mW. When the output of the pulse laser is less than 1 mJ/pulse and the output of the continuous wave laser is less than 10 mW, the lasers cannot transmit enough energy to disrupt cells, which may be a problem.

In the apparatus according to the current embodiment of the present invention, the laser may generate light in a wavelength range of more than 400 nm, and preferably in the range from 750 nm-1,300 nm. When the beam of the laser has a wavelength of less than 400 nm, DNA can be denatured or damaged., Further, the laser can be generate a laser beam having at least one wavelength range, that is, the laser may generate radiation have one wavelength within the wavelength range described above, and may have more than two different wavelengths.

Hereinafter, the present invention will be described in further detail with reference to the following examples. These examples are for illustrative purposes only and are not intended to limit the scope of the present invention.

### Examples

### Example 1: Manufacture of chip on which metal oxide is deposited

A chip on which metal oxide was deposited was manufactured by plasma assisted deposition (PAD), which is a PVD method, and by ALD. First, pillars were formed on the surface of a solid support by photolithography and dry etching. These processes will now be described in detail. A 4-inch wafer was coated with positive photoresist, (Model: AZ1512) using a spin coating method at 4000 RPM for 40 seconds, and then baked at 95 °C for 1.5 minutes. In an aligner (Model: EV620), I-line UV light (wavelength: 365 nm) was irradiated to the positive photoresist-coated wafer for 4.5 seconds, and then the positive photoresist that was exposed to the UV light was removed using a developer (Model: AZ MIF 300) resulting in a pillar pattern. The obtained wafer having the pillar pattern was dry etched to a depth of 100 µm using a dry etching apparatus (Model: ICP-STS) to finally form rectangular parallelepiped pillars each having a size of 25 µm x 25 µm x 100 µm (width x length x height). Fe₂O₃ metal oxide used as a cell lysis enhancing layer was deposited to a depth of 300 nm on the rectangular parallelepiped pillar surfaces using a plasma assisted deposition method (PAD, model: APS1104). Subsequently, a glass plate having pores of 1 mm diameter was coupled to the chip having the pillars using an anodic binding apparatus (Model: TPS-1000) at 370 °C while 700 V was applied. Then, a metal oxide, Al₂O₃, used as a cell binding and gene binding layer was deposited on the chip to a depth of 100A at 400 °C using an ALD apparatus (atomic layer deposition, Model: MOOHAN) to complete the chip.

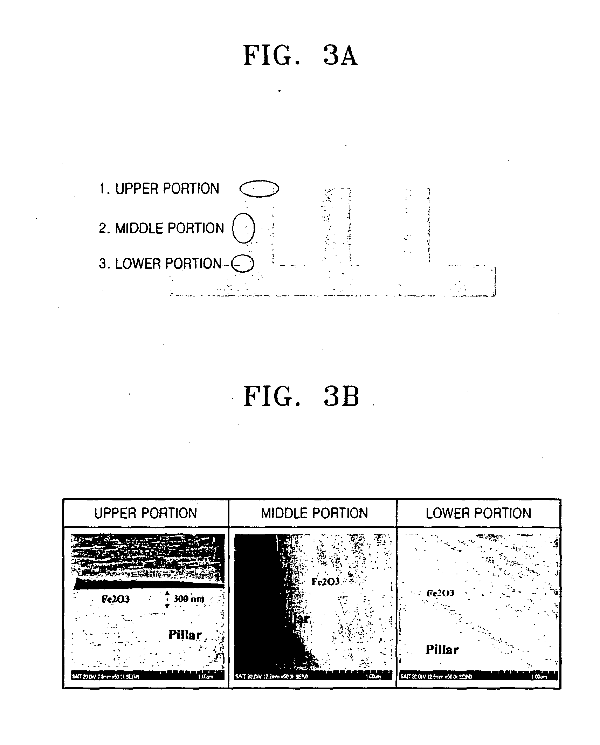

FIG. 3A illustrates a top portion, a middle portion and a lower portion of a chip on which metal oxide used as a cell lysis enhancing layer was deposited, and FIG. 3B is a series of field emission-scanning electron microscope (FE-SEM) images of the chip taken at each of the positions described above. As can be seen in FIG. 3B, a Fe₂O₃ deposition film having a depth of 100 µm is formed up to the inside of the pillar, so that the cell lysis enhancing metal oxide is considered to be deposited satisfactorily.

### Example 2: Cell binding using the chip

It was determined if a cell was bound to a chip according to an embodiment of the present invention in a fluidic control system. First, a mixture of saliva filtered using a filter paper (pore size 6 µm, Whatman) and *E. coli* BL21 (Stratagen Cat. 200133) cells was prepared as a cell-containing sample. 680 µℓ of a mixture comprising the sample (cell concentration:OD₆₀₀ = 0.001) and a binding buffer (100 mM phosphate buffer, pH 4) in a ratio of 1:1 was injected to the chip at a flow rate of 400 µℓ/min using a syringe pump (HARVARD, PHD2000), in order to bind *E. coli* cells to the chip.

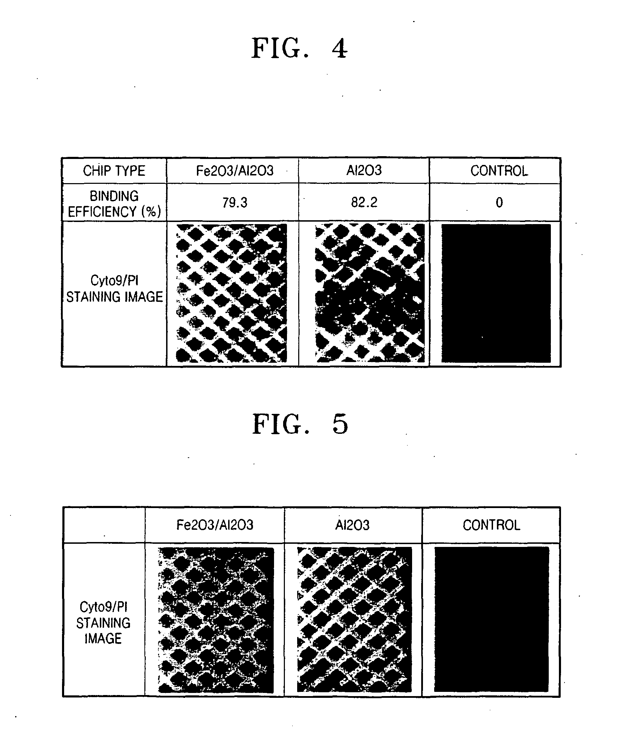

FIG. 4 is a series of FE-SEM images showing a binding efficiency of *E*. *coli* cells according to different kinds of chips. In FIG. 4, a Fe₂O₃/Al₂O₃ chip uses Fe₂O₃ as a cell lysis enhancing metal oxide, and uses Al₂O₃ as a metal oxide that can bind cells. In FIG. 4, an Al₂O₃ chip uses only Al₂O₃ as a metal oxide, and a control uses a Fe₂O₃/Al₂O₃ chip through which only binding buffer without *E. coli* cells was passed. A plating method was used for determining the binding efficiency of *E. coli.* The binding efficiency was measured by plating a solution before and after being passed through the chip in a petri dish containing *E. coli* growth medium. Whether a cell was bound to the chip according to the present invention was determined using LIVE/DEAD^{®} BacLight^{™} Bacterial viability kit (manufactured by Molecular probe). A living cell was stained green by Cyta^{®}9 nucleic acid stain dye included in LIVE/DEAD^{®} BacLight^{™} Bacterial viability kit, and a dead cell was stained red by PI(Propidium Iodide) dye.

As can be seen in FIG. 4, in case of Cyto9/PI staining, the living *E. coli* bound to the Fe₂O₃/Al₂O₃ chip and the Al₂O₃ chip, thereby showing a green image as expected, and the control chip does not show a green image due to cell absence. Also, the efficiency of binding *E. coli* to the Fe₂O₃/Al₂O₃ chip and the Al₂O₃ chip is about 80% in both cases, so that *E. coli* can be considered being efficiently bound to the chip.

### Example 3: Cell lysis using the chip

A cell lysis test was performed by irradiating a laser beam to the cells bound to the chip of Example 2. The cells were lysed by irradiating a 2 W laser beam to a Fe₂O₃/Al₂O₃ chip, an Al₂O₃ chip and a control chip for 40 seconds. After cell lysis, staining was performed using LIVE/DEAD^{®} BacLight^{™} Bacterial viability kit. FIG. 5 is a FE-SEM image showing *E. coli* cell lysis according to the different kinds of chips used. As can be seen in FIG. 5, the Fe₂O₃/Al₂O₃ chip shows a higher intensity of red color compared to the Al₂O₃ chip. The control chip does not show a red image due to cell absence. Accordingly, it can be seen that cells bound to the Fe₂O₃/Al₂O₃chip according to an embodiment of the present invention can be efficiently lysed, and also cell lysis can be more efficiently performed when a cell lysis enhancing metal oxide (Fe₂O₃ in the present example) is used compared to when it is not used.

### Example 4: Nucleic acid purification using the chip

An experiment of purifying the nucleic acid released from the lysed cell in Example 3 was performed. To remove impurities excluding *E*. *coli* genome DNA from the cell lysate after lysis by laser irradiation, a washing buffer (10mM phosphate buffer, pH 4) was injected to the chip at a flow rate of 100 µℓ/min using a syringe pump (HARVARD, PHD2000). Subsequently, to elute the washed nucleic acid from the chip, a nucleic acid eluting buffer (50 mM Na₂SO₄, pH 10) was injected to the chip at a flow rate of 1000 µl/min using a syringe pump (HARVARD, PHD2000).

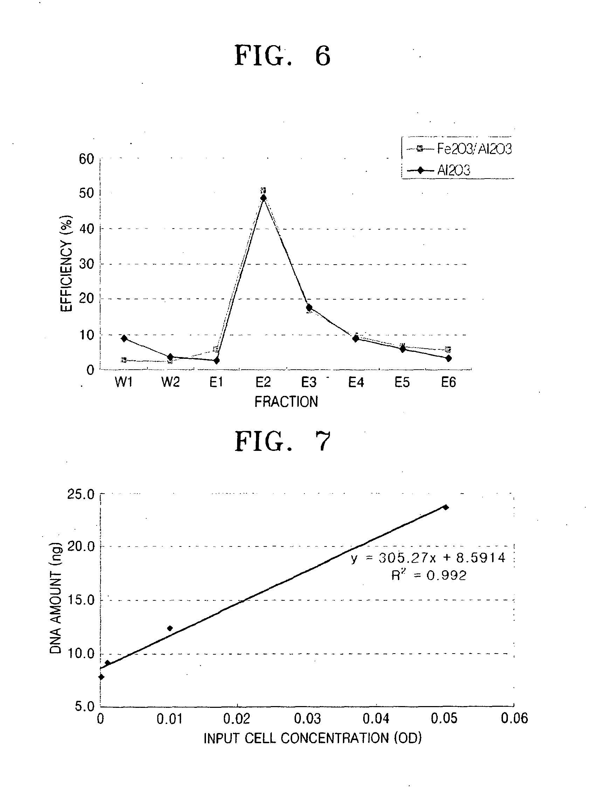

FIG. 6 is a graph showing the efficiency of eluting *E. coli* genome DNA (gDNA) applying the washing buffer and a nucleic acid eluting buffer. In FIG. 6, W1 and W2 each refer to 25 µℓ fraction of the washing buffer, E1 through E6 each refer to 25 *µℓ* fraction of the nucleic acid eluting buffer. The eluting efficiency corresponds to the amount of *E. coli* genome DNA eluted in each fraction with respect to the total amount of *E. coli* genome DNA eluted. As can be seen in FIG. 6, while *E. coli* genome DNA is hardly eluted using the washing buffer in the case of a Fe₂O₃/Al₂O₃ chip and a Al₂O₃ chip, most of the *E. coli* genome DNAs is eluted in the nucleic acid eluting buffer. As a result, it can be seen that nucleic acid can be efficiently isolated and purified after a cell is lysed, all in one single chamber, when the method according to an embodiment the present invention is used.

### Example 5: Cell enrichment using the chip

Cell enrichment was determined using the chip according to an embodiment of the present invention. Using the method of purifying *E. coli* genome DNA from *E. coli* according to Example 4, the amount of purified *E. coli* genome DNAs with respect to the concentration of *E. coli* input were quantified. A method of measuring cell enrichment was performed such that firstly *E. coli* solutions with known *E. coli* cell concentrations determined in an Eppendorf tube were lysed by laser irradiation and then the DNA released was quantified using picogreen. Based on this, a quantification curve of DNA eluted after lysis depending on the cell concentration before the lysis was drawn. On the basis of this quantification curve, the amount of DNA eluted from the chip according to an embodiment of the present invention was converted to the corresponding cell concentration to calculate enrichment efficiency.

FIG. 7 is a graph illustrating a quantification curve of eluted DNA over the input *E. coli* cell concentration. As can be seen in FIG. 7, input *E. coli* cell concentration is nearly proportional to the amount of the *E. coli* genome DNA determined. The purified *E. coli* genome DNA using the chip according to an embodiment of the present invention was converted to the cell concentration using the quantification curve of FIG. 7, and the results are shown in Table 1 below.

**Table 1**

| | Fe₂O₃/Al₂O₃ chip | Al₂O₃chip |
|---|---|---|
| Input cell concentration(OD) | 0.001 | 0.001 |
| Output cell concentration(OD) | 0.014 | 0.016 |
| Enrichment ratio | 14 times | 16 times |

As can be seen in Table 1, *E. coli* cells are enriched from up to 14 times to 16 times when the chip according to an embodiment of the present invention is used. Therefore, when nucleic acids from a test sample having a very low cell concentration shall be detected, the method according to the present invention can be effectively used.

### Example 6: PCR amplification of nucleic acid purified using the chip

PCR was performed using the nucleic acid purified using the chip according to an embodiment of the present invention. PCR was performed under a standard condition known to those skilled in the art. Using the method of purifying *E*. *coli* genome DNA from *E*. *coli* cells according to Example 4, a PCR amplification product of *E. coli* genome DNA was purified with respect to concentration of the measured input *E. coli* cells. A method of measuring cell enrichment was performed such that firstly *E. coli* cell solutions with known *E. coli* cell concentrations determined in an Eppendorf tube were lysed by laser irradiation , then the *E. coli* genome DNA released was amplified by PCR and quantified using Labship. Based on this, a quantification curve of the PCR product after lysis with respect to the input cell concentration was drawn, and then an amount of the PCR product obtained using the chip according to the present invention was converted to cell concentration to calculate enrichment efficiency.

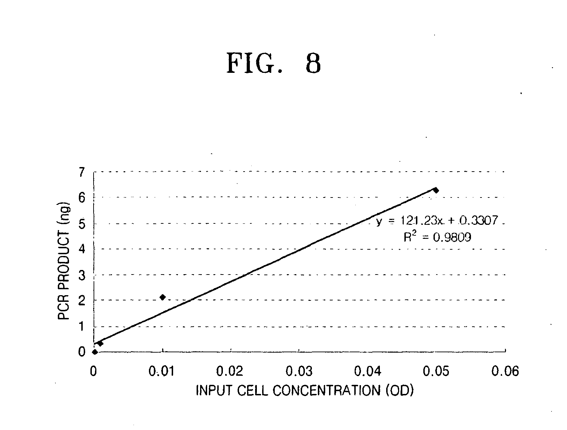

FIG. 8 is a graph illustrating the quantification curve of PCR product over the input cell concentration of *E. coli.* As can be seen in FIG. 8, the input cell concentration of *E. coli* is nearly proportional to an amount of the PCR product. The PCR product using the chip according to an embodiment of the present invention was converted to the corresponding cell concentration using the quantification curve of FIG. 8, and the results are shown in Table 2 below.

**Table 2**

| | Fe₂O₃/Al₂O₃ chip | Al₂O₃ chip |
|---|---|---|
| Input cell concentration(OD) | 0.001 | 0.001 |
| PCR product amount (ng) | 0.88 | 0.65 |
| Output cell concentration(OD) | 0.005 | 0.003 |
| Enrichment ratio | 5 times | 3 times |

As can be seen in Table 2, *E. coli* cells are concentrated from up to 3 times to 5 times when the chip according to an embodiment of the present invention is used. The result described above is different from result of the cell enrichment shown in Table 1, but the difference is considered to be a difference in the method for quantifying DNA used.

According to the present invention, by developing a chip wherein cell enrichment, cell lysis, and nucleic acid purification are be carried out in a single chamber, integration of a Lab-On-a-Chip can be achieved, and the three processes can be performed on one chip so that the cost of manufacturing the chip can be reduced.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A method of disrupting cells or viruses and purifying nucleic acids, the method comprising:
adding a cell-containing or virus-containing solution to a solid support onto which a lysis enhancement metal oxide capable of enhancing cell or virus lysis and a binding metal oxide capable of binding a) cells or viruses, and b) nucleic acid are sequentially deposited for binding the cells or viruses to the deposited binding metal oxide, wherein the lysis enhancement metal oxide is Fe₂O₃ and the binding metal oxide is Al₂O₃;
irradiating a laser beam onto the binding metal oxide for disrupting the cells or viruses bound to the binding metal oxide, whereupon nucleic acids released from the lysed cells or viruses are bound to the binding metal oxide; and
washing off the unbound cell or virus components using a washing buffer.

2. The method according to claim 1, further comprising the step of eluting the nucleic acids bound to the binding metal oxide by adding a nucleic acid eluting buffer.

3. The method according to claim 1, wherein the solid support is selected from the group consisting of slide glass, a silicon wafer, a magnetic bead, polystyrene, a membrane and a metal plate.

4. The method according to claim 1, wherein the cell-containing or virus-containing solution and the washing buffer have a pH of 3-10.

5. The method according to claim 1, wherein the cell-containing or virus-containing solution, the washing buffer and the nucleic acid eluting buffer are selected from the group consisting of phosphate buffer, acetate buffer, citrate buffer and Tris buffer.

6. The method according to claim 1, wherein the cell-containing or virus-containing solution and the washing buffer have a concentration of 10-1,000 mM.

7. The method according to claim 2, wherein the nucleic acid eluting buffer has a pH of 8-10.

8. The method according to claim 2, wherein the nucleic acid eluting buffer has a concentration of 10-100 mM.

9. The method according to claim 2, further comprising amplifying the eluted nucleic acid after eluting the nucleic acids from the solid support.

10. The method according to claim 9, wherein amplifying the nucleic acids is performed in the presence of the nucleic acid eluting buffer.

11. The method according to any of the above mentioned claims, further comprising detecting the eluted nucleic acids after eluting the nucleic acids from the solid support.

12. The method according to claim 11, wherein the eluted nucleic acids are detected after amplifying the nucleic acids.

13. The method according to claim 1, wherein the laser beam is irradiated as a laser pulse or a continuous laser wave.

14. The method of claim 13, wherein the laser pulse has an output of more than 1 mJ/pulse and the continuous laser wave has an output of more than 10 mW.

15. A chip for disrupting cells or viruses and purifying nucleic acids comprising a solid support onto which a lysis enhancement metal oxide enhancing cell or virus lysis and a binding metal oxide binding a) cells or viruses, and b) nucleic acid have been sequentially deposited, wherein the lysis enhancement metal oxide is Fe₂O₃ and the binding metal oxide is Al₂O₃.

16. The chip according to claim 15, wherein the solid support has a planar or pillar structure.

17. The chip according to claim 15, wherein lysis enhancement metal oxide has been deposited onto the solid substrate and the binding metal oxide has been deposited on the lysis enhancement metal oxide.

18. An apparatus for disrupting cells and purifying nucleic acids, the apparatus comprising:
a chip according to any of claims 15 to 17; and
a laser generating means.

19. The apparatus according to claim 18, wherein the laser is a pulse laser or a continuous wave laser.

20. The apparatus according to claim 19, wherein the pulse laser has an output of more than 1 mJ/pulse, and the continuous wave laser has an output of more than 10 mW.

21. A method for manufacturing a chip for disrupting cells or viruses and purifying nucleic acids according to claim 15, the method comprising sequentially depositing on a solid support a lysis enhancement metal oxide capable of enhancing cell or virus lysis and a binding metal oxide capable of binding a) cells or viruses, and b) nucleic acid, wherein the lysis enhancement metal oxide is Fe₂O₃ and the binding metal oxide is Al₂O₃.

## Patentansprüche

1. Verfahren zum Aufschließen von Zellen oder Viren und zum Aufreinigen von Nukleinsäuren, wobei das Verfahren umfasst:
Zugeben einer Zellen- oder Viren-enthaltenden Lösung zu einem festen Träger, auf dem ein die Zelllyse verstärkendes Metalloxid, das zur Verbesserung der Lyse von Zellen oder Viren in der Lage ist, und ein Bindungsmetalloxid, das dazu in der Lage ist, a) Zellen oder Viren, und b) Nukleinsäure zu binden, sequentiell aufgetragen sind, um Zellen oder Viren an das aufgetragene Bindungsmetalloxid zu binden, wobei das die Zelllyse verstärkende Metalloxid Fe₂O₃ ist und das Bindungsmetalloxid Al₂O₃ ist;
Bestrahlen eines Laserstrahls auf das Bindungsmetalloxid zum Aufschließen der Zellen oder Viren, die an das Bindungsmetalloxid gebunden sind, woraufhin Nukleinsäuren, die aus den lysierten Zellen oder Viren freigesetzt wurden, an das Bindungsmetalloxid gebunden werden; und
Auswaschen der ungebundenen Zell- oder Virenbestandteile mit einem Waschpuffer.

2. Verfahren gemäß Anspruch 1, das ferner den Schritt des Eluierens der Nukleinsäuren, die an das Bindungsmetalloxid gebunden sind, durch Zugeben eines Nukleinsäure-Elutionspuffers umfasst.

3. Verfahren gemäß Anspruch 1, wobei der feste Träger ausgewählt ist aus der Gruppe bestehend aus einem Objektträger, einer Siliziumscheibe, einem magnetischen Kügelchen, Polystyrol, einer Membran und einer Metallplatte.

4. Verfahren gemäß Anspruch 1, wobei die Zellen- oder Viren-enthaltende Lösung und der Waschpuffer einen pH von 3-10 aufweisen.

5. Verfahren gemäß Anspruch 1, wobei die Zellen- oder Viren-enthaltende Lösung, der Waschpuffer und der Nukleinsäure-Elutionspuffer ausgewählt werden aus der Gruppe bestehend aus Phosphatpuffer, Acetatpuffer, Citratpuffer, und Tris-Puffer.

6. Verfahren gemäß Anspruch 1, wobei die Zellen- oder Viren-enthaltende Lösung und der Waschpuffer eine Konzentration von 10 - 1000 mM ausweisen.

7. Verfahren gemäß Anspruch 2, wobei der Nukleinsäure-Elutionspuffer einen pH von 8 - 10 aufweist.

8. Verfahren gemäß Anspruch 2, wobei der Nukleinsäure-Elutionspuffer eine Konzentration von 10 - 100 mM aufweist.

9. Verfahren gemäß Anspruch 2, ferner umfassend das Amplifizieren der eluierten Nukleinsäure nach dem Eluieren der Nukleinsäuren von dem festen Träger.

10. Verfahren gemäß Anspruch 9, wobei das Amplifizieren der Nukleinsäuren in der Gegenwart von dem Nukleinsäure-Elutionspuffer durchgeführt wird.

11. Verfahren gemäß einem der oben genannten Ansprüche, ferner umfassend das Detektieren der eluierten Nukleinsäuren nach dem Eluieren der Nukleinsäuren von dem festen Träger.

12. Verfahren gemäß Anspruch 11, wobei die eluierten Nukleinsäuren nach dem Amplifizieren der Nukleinsäuren detektiert werden.

13. Verfahren gemäß Anspruch 1, wobei der Laserstrahl als Laserpuls oder als kontinuierlicher Laserstrahl ausgestrahlt wird.

14. Verfahren gemäß Anspruch 13, wobei der Laserpuls eine Leistung von mehr als 1 mJ/Puls und der kontinuierliche Laserstrahl eine Leistung von mehr als 10 mW aufweist.

15. Chip zum Aufschluss von Zellen oder Viren und zur Aufreinigung von Nukleinsäuren, umfassend einen festen Träger auf dem ein die Lyse verstärkendes Metalloxid, das die Lyse von Zellen oder Viren verbessert, und ein Bindungsmetalloxid, das a) Zellen oder Viren, und b) Nukleinsäure bindet, die nacheinander aufgetragen sind, wobei das die Lyse verstärkende Metalloxid Fe₂O₃ ist und das Bindungsmetalloxid Al₂O₃ ist.

16. Chip gemäß Anspruch 15, wobei der feste Träger eben ist oder eine Säulenstruktur aufweist.

17. Chip gemäß Anspruch 15, wobei das die Lyse verstärkende Metalloxid auf dem festen Träger aufgebracht ist und das Bindungsmetalloxid auf dem die Lyse verstärkenden Metalloxid aufgebracht ist.

18. Vorrichtung zum Aufschluss von Zellen und zur Aufreinigung von Nukleinsäuren, wobei die Vorrichtung umfasst:
einen Chip gemäß einem der Ansprüche 15-17; und
einen Lasergenerator.

19. Vorrichtung gemäß Anspruch 18, wobei der Laser ein Pulslaser oder ein Dauerstrichlaser ist.

20. Vorrichtung gemäß Anspruch 19, wobei der Pulslaser eine Leistung von mehr als 1 mJ/puls und der Dauerstrichlaser eine Leistung von mehr als 10 mW aufweist.

21. Verfahren zum Herstellen eines Chips zum Aufschluss von Zellen oder Viren und zur Aufreinigung von Nukleinsäuren gemäß Anspruch 15, wobei das Verfahren das aufeinander folgende Auftragen eines die Lyse verstärkenden Metalloxids, das zur Verstärkung der Lyse von Zellen oder Viren in der Lage ist, und eines Bindungsmetalloxids, das dazu in der Lage ist, a) Zellen oder Viren und b) Nukleinsäure zu binden, auf einen festen Träger umfasst, wobei das die Lyse verstärkende Metalloxid Fe₂O₃ ist und das Bindungsmetalloxid Al₂O₃ ist.

## Revendications

1. Procédé de dissociation de cellules ou de virus et de purification d'acides nucléiques, ledit procédé consistant à :
ajouter une solution contenant des cellules ou contenant des virus à un support solide sur lequel un oxyde métallique activateur de lyse capable d'activer la lyse cellulaire ou virale et un oxyde métallique de liaison capable de lier a) des cellules ou des virus, et b) de l'acide nucléique, sont déposés successivement, afin de lier les cellules ou les virus à l'oxyde métallique de liaison déposé, l'oxyde métallique activateur de lyse étant Fe₂O₃ et l'oxyde métallique de liaison étant Al₂O₃ ;
irradier l'oxyde métallique de liaison avec un faisceau laser afin de dissocier les cellules ou les virus liés à l'oxyde métallique de liaison, après quoi les acides nucléiques libérés par les cellules ou virus lysés sont liés à l'oxyde métallique de liaison ; et
éliminer par lavage les constituants cellulaires ou viraux non liés, à l'aide d'un tampon de lavage.

2. Procédé selon la revendication 1, comprenant en outre l'étape d'élution des acides nucléiques liés à l'oxyde métallique de liaison, par addition d'un tampon d'élution d'acide nucléique.

3. Procédé selon la revendication 1, dans lequel le support solide est choisi dans le groupe constitué d'un verre pour lame, d'une plaquette de silicium, d'une bille magnétique, de polystyrène, d'une membrane et d'une plaque métallique.

4. Procédé selon la revendication 1, dans lequel la solution contenant des cellules ou contenant des virus et le tampon de lavage ont un pH de 3-10.

5. Procédé selon la revendication 1, dans lequel la solution contenant des cellules ou contenant des virus, le tampon de lavage et le tampon d'élution d'acide nucléique sont choisis dans le groupe constitué d'un tampon phosphate, d'un tampon acétate, d'un tampon citrate et d'un tampon Tris.

6. Procédé selon la revendication 1, dans lequel la solution contenant des cellules ou contenant des virus et le tampon de lavage ont une concentration de 10-1000 mM.

7. Procédé selon la revendication 2, dans lequel le tampon d'élution d'acide nucléique a un pH de 8-10.

8. Procédé selon la revendication 2, dans lequel le tampon d'élution d'acide nucléique a une concentration de 10-100 mM.

9. Procédé selon la revendication 2, comprenant en outre l'amplification de l'acide nucléique élué, après l'élution des acides nucléiques du support solide.

10. Procédé selon la revendication 9, dans lequel l'amplification des acides nucléiques est effectuée en présence du tampon d'élution d'acide nucléique.

11. Procédé selon l'une quelconque des revendications précédentes, comprenant en outre la détection des acides nucléiques élués, après l'élution des acides nucléiques du support solide.

12. Procédé selon la revendication 11, dans lequel les acides nucléiques élués sont détectés après l'amplification des acides nucléiques.

13. Procédé de la revendication 1, dans lequel le faisceau laser irradie sous forme d'impulsion laser ou sous forme d'onde laser continue.

14. Procédé selon la revendication 13, dans lequel l'impulsion laser a une énergie de sortie supérieure à 1 mJ/impulsion et l'onde laser continue a une puissance de sortie supérieure à 10 mW.

15. Puce permettant la dissociation de cellules ou de virus et la purification d'acides nucléiques comprenant un support solide sur lequel un oxyde métallique activateur de lyse activant la lyse cellulaire ou virale et un oxyde métallique de liaison liant a) des cellules ou des virus, et b) de l'acide nucléique, ont été déposés successivement, l'oxyde métallique activateur de lyse étant Fe₂O₃ et l'oxyde métallique de liaison étant Al₂O₃.

16. Puce selon la revendication 15, dans laquelle le support solide a une structure plane ou une structure de type pilier.

17. Puce selon la revendication 15, dans laquelle l'oxyde métallique activateur de lyse a été déposé sur le substrat solide et l'oxyde métallique de liaison a été déposé sur l'oxyde métallique activateur de lyse.

18. Appareil servant à dissocier des cellules et à purifier des acides nucléiques, ledit appareil comprenant :
une puce selon l'une quelconque des revendications 15 à 17 ; et
un moyen de génération de laser.

19. Appareil selon la revendication 18, dans lequel le laser est un laser à impulsions ou un laser à onde continue.

20. Appareil selon la revendication 19, dans lequel le laser à impulsions a une énergie de sortie supérieure à 1 mJ/impulsion et le laser à onde continue a une puissance de sortie supérieure à 10 mW.

21. Procédé de fabrication d'une puce permettant la dissociation de cellules ou de virus et la purification d'acides nucléiques selon la revendication 15, ledit procédé consistant à déposer successivement sur un support solide un oxyde métallique activateur de lyse capable d'activer la lyse cellulaire ou virale et un oxyde métallique de liaison capable de lier a) des cellules ou des virus, et b) de l'acide nucléique, l'oxyde métallique activateur de lyse étant Fe₂O₃ et l'oxyde métallique de liaison étant Al₂O₃.
